# EUROPÄISCHE PATENTSCHRIFT

(11) **EP 2 598 052 B1**
(45) Veröffentlichungstag und Bekanntmachung des Hinweises auf die Patenterteilung: **14.03.2018**
(21) Anmeldenummer: 11730691.0
(22) Anmeldetag: 12.07.2011
(51) Int. Cl.: A61B 17/076

(54) **CHIRURGISCHES INSTRUMENT ZUM ENTFERNEN CHIRURGISCHER CLIPS**
SURGICAL INSTRUMENT FOR REMOVING SURGICAL CLIPS
INSTRUMENT CHIRURGICAL POUR ENLEVER LES CLIPS CHIRURGICAUX

(30) Priorität: 28.07.2010 DE 102010036713
(43) Veröffentlichungstag der Anmeldung: 05.06.2013
(73) Patentinhaber: Aesculap AG, 78532 Tuttlingen (DE)
(72) Erfinder: MORALES, Pedro, 78532 Tuttlingen-Nendingen (DE)
(74) Vertreter: Hoeger, Stellrecht & Partner Patentanwälte mbB
(86) Internationale Anmeldenummer: PCT/EP2011/061898
(87) Internationale Veröffentlichungsnummer: WO 2012/013494

(56) Entgegenhaltungen:
- WO-A1-96/16602
- WO-A2-2007/009099
- US-A- 439 994
- US-A- 5 752 973
- US-A1- 2004 044 363
- US-A1- 2005 119 677

## Beschreibung

Die vorliegende Erfindung betrifft ein chirurgisches Instrument zum Entfernen eines an ein Hohlorgan angelegten chirurgischen Clips, welcher zwei Klemmarme aufweist, die zwei freie Enden und zwei miteinander verbundene, einen Verbindungsbereich definierende Enden aufweisen, welches Instrument ein proximales und ein distales Ende aufweist, wobei am distalen Ende eine Spreizeinrichtung zum Fassen und Aufspreizen eines angelegten Clips von einer Anlegestellung in eine Lösestellung angeordnet ist, wobei die Spreizeinrichtung eine Clipaufnahme umfasst und wobei sie von einer Einführstellung, in welcher sie eine minimale Querschnittsfläche definiert, in eine Clipaufnahmestellung überführbar ist, in welcher die Clipaufnahme zur Aufnahme des zu entfernenden Clips geöffnet ist, wobei die Spreizeinrichtung mindestens einen proximalen und mindestens einen distalen Clipaufnahmeanschlag aufweist, welche die Clipaufnahme proximalseitig und distalseitig begrenzen, und wobei zwei distalseitige Clipaufnahmeanschläge vorgesehen sind.

In der Chirurgie kommen chirurgische Clips, insbesondere in Form von sogenannten Ligaturclips, vermehrt zur Blutstillung von Blutgefäßen sowie zum Abklemmen und Verschließen anderer Hohlorgane zum Einsatz. Die Clips können zwei im Wesentlichen parallel verlaufende, zwei freie Enden und zwei miteinander verbundene Enden und einen Verbindungsbereich aufweisende Klemmarme umfassen. Auch zum Einsatz kommen beispielsweise sogenannte Doppelstegclips, welche insgesamt vier Klemmarme mit vier freien Enden, die paarweise verbunden sind, und vier ebenfalls paarweise miteinander verbundene und zwei Verbindungsbereiche definierende Enden umfassen. Mit solchen Doppelstegclips, die aus einem in sich geschlossenen Drahtring geformt werden können, lassen sich Gefäße oder Hohlorgane auf einfache Weise sogar an zwei voneinander getrennten Stellen verschließen.

Das Setzen von Clips ist deutlich einfacher und schneller als bisher verwandte Nahttechniken. Die Clips sind, da sie normalerweise als Implantat im Körper verbleiben, aus biokompatiblen Materialien hergestellt, insbesondere aus reinem Titan oder einer Titanlegierung.

Bevor bei einer Operation ein Blutgefäß oder ein Hohlorgan durchtrennt werden kann, wird dieses üblicherweise mit mindestens zwei, besser noch mit drei Clips derart verschlossen, dass ein Operateur das Hohlorgan zwischen zwei gesetzten Clips durchtrennen kann, ohne dass Blut oder andere Körperflüssigkeiten austreten können.

Immer wieder kommt es vor, dass ein oder mehrere Clips falsch positioniert werden, beispielsweise an einem falschen Gefäß oder Hohlorgan. Sollte dieser Fall eintreten, müssen die Clips wieder entfernt werden, damit die ursprüngliche Funktion des Gefäßes beziehungsweise des Hohlorgans wiederhergestellt werden kann. In der sogenannten offenen Chirurgie ist das Entfernen falsch positionierter Clips beispielsweise mit Hilfe von Nadelhaltern, Pinzetten oder anderen Instrumenten meist noch möglich, wenn auch mit einem sehr großen Aufwand. Bei endoskopischen Eingriffen ist das falsche Positionieren eines Clips äußerst kritisch. Die Clips sind üblicherweise sehr klein und auch von ihrer Bauart so gestaltet, dass sie keine Hinterschnitte oder andere abstehende Komponenten aufweisen, an denen man diese gut fassen könnte. Dies ist auch nicht erwünscht, da im Idealfall beide Klemmarme des Clips, auch Clipschenkel genannt, oder jeweils zwei Klemmarme paarweise bei einem Doppelstegclip, deckungsgleich und ohne Spalt aufeinander liegen.

Aus der WO 96/16602 A1 ist ein Ligaturclipentferner bekannt. In der US 2004/0044363 A1 sind Vorrichtungen und Verfahren zum Entfernen von Clips beschrieben. Vorrichtungen und Verfahren zum Verschließen hohler anatomischer Strukturen sind in der WO 2007/009099 A2 offenbart. Aus der US 2005/0119677 A1 ist ein Ligaturclipapplikator bekannt. In der US 5,752,973 ist ein endoskopisches chirurgisches Greifinstrument bekannt. In der US 439,994 ist ein Instrument zum Öffnen von Bindeclips beschrieben.

Es ist daher eine Aufgabe der vorliegenden Erfindung, ein chirurgisches Instrument der eingangs beschriebenen Art so zu verbessern, dass chirurgische Clips nach dem Anlegen auf einfache Weise wieder entfernt werden können.

Diese Aufgabe wird bei einem chirurgischen Instrument der eingangs beschriebenen Art erfindungsgemäß dadurch gelöst, dass ein Querabstand zwischen den beiden distalseitigen Clipaufnahmeanschlägen quer zur Längsrichtung beim Übergang von der Clipaufnahmestellung in eine Clipspreizstellung, in welcher der zu entfernende Clip (12) in der Clipaufnahme (112) aufgespreizt haltbar ist, zunimmt und dass ein Abstand in Längsrichtung zwischen dem mindestens einen proximalen und dem mindestens einen distalen Clipaufnahmeanschlag veränderbar ist.

Wenn das Instrument beispielsweise in Form eines endoskopischen Instruments ausgebildet ist, kann damit auch durch einen kleinen Zugang ins Innere eines Patientenkörpers ein zu entfernender Clips mit der Spreizeinrichtung auf einfache Weise gefasst und aufgespreizt werden, um ihn schonend und sicher zu entfernen, beispielsweise von einem Blutgefäß oder Hohlorgan, wo er insbesondere fälschlicherweise positioniert wurde. Die Spreizeinrichtung ist insbesondere derart ausgebildet, dass weitere Instrumente zur Entfernung des Clips nicht erforderlich sind. Vorzugsweise kann der Clip mit der Spreizeinrichtung automatisch entfernt werden, vorzugsweise ist er mit dem Instrument nach dem Aufspreizen sicher haltbar und kann durch einen insbesondere minimalinvasiven Zugang zum menschlichen Körper, beispielsweise einen Trokar, wieder entfernt werden. Vorteilhaft ist es, dass die Spreizeinrichtung eine Clipaufnahme umfasst und dass sie von einer Einführstellung, in welcher sie eine minimale Querschnittsfläche definiert, in eine Clipaufnahmestellung überführbar ist, in welcher die Clipaufnahme zur Aufnahme des zu entfernenden Clips geöffnet ist. Die Clipaufnahme ermöglicht es, den Clip sicher zu fassen. In der Einführstellung benötigt die Spreizeinrichtung besonders wenig Platz, so dass sie auch durch einen kleinen, insbesondere minimalinvasiven Zugang ins Innere eines Patientenkörpers eingeführt werden kann. In der Clipaufnahmestellung kann der Clip in die Clipaufnahme eingeführt beziehungsweise in dieser aufgenommen werden. Um eine Bewegung des Clips relativ zur Spreizeinrichtung zu verhindern und ihn mit dieser sicher fassen und halten zu können, ist es vorteilhaft, dass die Spreizeinrichtung mindestens einen proximalen und mindestens einen distalen Clipaufnahmeanschlag aufweist, welche die Clipaufnahme proximalseitig und distalseitig begrenzen. Insbesondere können die Clipaufnahmeanschläge so ausgebildet sein, dass auch eine Bewegung quer zu einer Verbindungsrichtung zwischen proximalen und distalen Clipaufnahmeanschlägen nicht möglich ist. So kann verhindert werden, dass der Clip aus der Clipaufnahme herausfallen kann, wenn er proximalseitig und distalseitig an den jeweiligen proximalen und distalen Clipaufnahmeanschlägen anliegt. Günstigerweise sind zwei distalseitige Clipaufnahmeanschläge vorgesehen. Insbesondere kann jeder der zwei distalseitigen Clipaufnahmeanschläge angepasst sein in seiner Form an eines der zwei freien Enden der Klemmarme eines einfachen Clips oder an einen der zwei Verbindungsbereiche eines Doppelstegclips. Günstig ist es, dass ein Querabstand zwischen den beiden distalseitigen Clipaufnahmeanschlägen quer zur Längsrichtung beim Übergang von der Clipaufnahmestellung in die Clipspreizstellung zunimmt. Durch eine Bewegung der distalseitigen Clipaufnahmeanschläge voneinander weg können freie Enden der Klemmarme eines einfachen Clips oder in einer Anlegestellung aneinander anliegende Verbindungsbereiche eines Doppelstegclips, wenn sie an den jeweiligen distalen Clipaufnahmeanschlägen anliegen, auf einfache Weise voneinander weg bewegt werden, so dass der Clip ein Gefäß oder Hohlorgan freigeben kann. Günstig ist es ferner, dass die Spreizeinrichtung eine Clipaufnahme umfasst, und dass sie von einer Clipaufnahmestellung, in welcher die Clipaufnahme zur Aufnahme des zu entfernenden Clips geöffnet ist, in eine Clipspreizstellung überführbar ist, in welcher der zu entfernende Clip in der Clipaufnahme aufgespreizt haltbar ist. Eine so ausgebildete Spreizeinrichtung ermöglicht es, einen Clip aufzunehmen und ihn, so lange er in der Clipaufnahme gehalten ist, aufzuspreizen. Durch Aufspreizen kann er von einem Gefäß oder Hohlorgan wieder gelöst werden, insbesondere indem die bei einem applizierten einfachen Clip im Wesentlichen aneinander anliegenden Klemmarme durch Verformung des Verbindungsbereichs aufgespreizt werden durch voneinander weg Schwenken. Bei einem Doppelstegclip können insbesondere die beiden in einer Anlege- oder Schließstellung aneinander anliegenden Verbindungsbereiche voneinander weg aufgespreizt werden. Um das Aufspreizen eines Clips zu erleichtern, ist es günstig, dass ein Abstand in Längsrichtung zwischen dem mindestens einen proximalen und dem mindestens einen distalen Clipaufnahmeanschlag veränderbar ist. Auf diese Weise kann insbesondere auch ein Abstand zwischen freien Enden der Klemmarme des zu entfernenden Clips und dessen Verbindungsbereich oder dessen Verbindungsbereichen verändert werden, was zum Beispiel zum Aufspreizen des Clips genutzt werden kann.

Gemäß einer weiteren bevorzugten Ausführungsform der Erfindung kann vorgesehen sein, dass die Spreizeinrichtung eine Clipaufnahme umfasst und dass sie von einer Clipspreizstellung, in welcher der zu entfernende Clip in der Clipaufnahme aufgespreizt gehalten ist, in eine Clipentnahmestellung überführbar ist, in welcher der zu entfernende Clip in der Clipaufnahme geschlossen haltbar ist. Die derart ausgebildete Spreizeinrichtung ermöglicht es insbesondere, den aufgespreizten Clip zu halten und, beispielsweise nachdem der Clip vom Gefäß oder Hohlorgan weg bewegt wurde, wieder zu schließen und in der geschlossenen Stellung zu halten. Dies erleichtert insbesondere das Herausführen des Clips aus dem Körper des Patienten durch einen minimalinvasiven Zugang. Grundsätzlich wäre es zwar denkbar, den Clip auch in der aufgespreizten Stellung aus einem Patientenkörper herauszubewegen. Allerdings würden hierfür deutlich größere Querschnitte der Zugänge benötigt.

Besonders einfach wird der Aufbau des Instruments, wenn die Clipentnahmestellung und die Einführstellung identisch sind. Dies gestattet es, das Instrument von der Einführstellung in die Clipaufnahmestellung zu überführen, den Clip aufzunehmen, anschließend den Clip durch Überführen des Instruments von der Clipaufnahmestellung in die Clipspreizstellung aufzuspreizen und schließlich den Clip nach Überführen des Instruments von der Clipspreizstellung in die Clipentnahmestellung, die der Einführstellung entspricht, aus dem Körper des Patienten herauszuführen.

Vorzugsweise sind der mindestens eine proximale und der mindestens eine distale Clipaufnahmeanschlag relativ zueinander bewegbar angeordnet. Beispielsweise können sie aufeinander zu bewegbar oder voneinander weg bewegbar ausgebildet sein, um bei in die Clipaufnahme aufgenommenem Clip, dessen freie Enden der Klemmarme relativ zu dem Verbindungsbereich oder den Verbindungsbereichen zu bewegen, beispielsweise um den Clip aufzuspreizen und gegebenenfalls wieder zu schließen.

Vorteilhafterweise nimmt der Abstand beim Übergang von der Clipaufnahmestellung in die Clipspreizstellung ab. Auf diese Weise ist es möglich, eine Spreizkraft in Längsrichtung auf den zu entfernenden Clip auszuüben, um freie Enden der Klemmarme eines einfachen Clips oder in einer Anlegestellung aneinander anliegende Verbindungsbereiche voneinander weg zu bewegen, um so ein Gefäß oder Hohlorgan freizugeben.

Zur Ausbildung insbesondere eines endoskopischen Instruments ist es günstig, wenn das Instrument einen langgestreckten Schaft umfasst, an dessen distalem Ende die Spreizeinrichtung angeordnet ist.

Die Konstruktion des Instruments wird besonders einfach, wenn der mindestens eine proximale Clipaufnahmeanschlag und der Schaft relativ zueinander unbeweglich angeordnet sind. Auf diese Weise lässt sich insbesondere ein Clip definiert in der Clipaufnahme aufnehmen, insbesondere indem der proximale Anschlag an den Verbindungsbereich eines einfachen Clips oder an freie Enden eines Doppelstegclips herangeführt und an diesen beziehungsweise diese angelegt wird.

Vorteilhaft ist, wenn der mindestens eine distale Clipaufnahmeanschlag und der Schaft relativ zueinander bewegbar angeordnet sind. Dies gestattet es, dass ein Operateur den Schaft mit dem proximalen Clipaufnahmeanschlag beispielsweise am Verbindungsbereich des Clips positioniert und ihn dort hält, wobei dann alle weiteren Bewegungen des mindestens einen distalen Clipaufnahmeanschlags relativ zum Schaft erfolgen. So ist ein Nachführen des Instrumentenschafts relativ zum Clip durch einen Operateur nicht erforderlich. Trotzdem sei angemerkt, dass es auch möglich wäre, beispielsweise den distalen Clipaufnahmeanschlag relativ zum Schaft unbewegbar anzuordnen und dagegen den mindestens einen proximalen Clipaufnahmeanschlag relativ zum Schaft bewegbar auszubilden.

Besonders einfach lässt sich ein Clip aufspreizen, wenn der mindestens eine distale Clipaufnahmeanschlag relativ zum Schaft um eine Schwenkachse verschwenkbar angeordnet ist. Beispielsweise kann so ein freies Ende eines Klemmarms relativ zum Verbindungsbereich oder zwei Verbindungsbereiche relativ zu den freien Enden der Klemmarme verschwenkt werden.

Günstigerweise verläuft die Schwenkachse quer zu einer vom Schaft definierten Längsachse. Dies erleichtert die Handhabung des Instruments, denn ein Clip kann so insbesondere parallel zu einer von ihm definierten Längsachse durch die Spreizeinrichtung aufgenommen und aufgespreizt werden.

Besonders kompakt ausbilden lässt sich die Spreizeinrichtung, wenn die beiden distalen Clipaufnahmeanschläge um eine gemeinsame Schwenkachse relativ zueinander verschwenkbar gelagert sind. Beispielsweise kann auch einer der beiden distalen Clipaufnahmeanschläge relativ zum Schaft feststehend angeordnet sein und nur einer relativ zum Schaft bewegbar, insbesondere verschwenkbar, ausgebildet sein.

Gemäß einer weiteren bevorzugten Ausführungsform der Erfindung kann vorgesehen sein, dass die Spreizeinrichtung zwei relativ zueinander bewegbare Werkzeugelemente umfasst und dass der mindestens eine distale Clipaufnahmeanschlag an den beiden Werkzeugelementen angeordnet oder ausgebildet ist. Insbesondere kann nur eines der beiden Werkzeugelemente mit einem distalen Clipaufnahmeanschlag ausgestattet sein. Die Werkzeugelemente können insbesondere eine Orientierung der Clipaufnahmeanschläge definieren und in der Einführstellung parallel oder aber auch quer zu einer Längsrichtung des Schafts orientiert sein.

Vorzugsweise umfasst jedes Werkzeugelement einen distalen Clipaufnahmeanschlag. Beispielsweise kann jeder distale Clipaufnahmeanschlag an ein freies Ende der Cliparme oder an einen Verbindungsbereich des Clips angelegt werden, um dann infolge einer Relativbewegung der Werkzeugelemente zueinander den zu entfernenden Clip aufzuspreizen.

Um nur möglichst wenig Gewebe um den zu entfernenden Clip herum freilegen zu müssen, ist es vorteilhaft, wenn der mindestens eine distale Clipaufnahmeanschlag im Bereich distaler Enden der Werkzeugelemente angeordnet oder ausgebildet ist.

Damit eine möglichst große Öffnung zum Aufnehmen eines zu entfernenden Clips in der Clipaufnahmestellung ermöglicht wird, ist es günstig, wenn die zwei Werkzeugelemente relativ zum Schaft bewegbar gelagert sind. Insbesondere kann so dann auch auf eine Bewegbarkeit des mindestens einen proximalen Clipaufnahmeanschlags relativ zum Schaft verzichtet werden.

Besonders einfach ausbilden lässt sich das Instrument, wenn die zwei Werkzeugelemente relativ zum Schaft verschiebbar und/oder verschwenkbar gelagert sind. Beispielsweise kann eine Schwenkachse für die zwei Werkzeugelemente quer, insbesondere senkrecht zu einer Längsrichtung des Schafts orientiert sein.

Gemäß einer weiteren bevorzugten Ausführungsform kann vorgesehen sein, dass die zwei Werkzeugelemente mit einem im oder am Schaft bewegbar gelagerten Kraftübertragungsglied beweglich gekoppelt sind zum Bewegen der Werkzeugelemente relativ zueinander infolge einer Bewegung des Kraftübertragungsglieds. Insbesondere kann so auf einfache Weise durch Bewegen des Kraftübertragungsglieds, beispielsweise eines Schub- und Zugglieds in Form einer Schub- und Zugstange, eine Bewegung der zwei Werkzeugelemente relativ zueinander erzwungen werden, beispielsweise eine Verschiebung und/oder eine Verschwenkung derselben relativ zueinander und gegebenenfalls auch relativ zum Schaft.

Grundsätzlich wäre es möglich, die Spreizeinrichtung lediglich in Verbindung mit einem Schaft oder einem kurzen Schaftabschnitt bereitzustellen. Vorteilhafterweise umfasst das Instrument jedoch auch eine Betätigungseinrichtung zum Betätigen der Spreizeinrichtung. Diese kann insbesondere mit dem Kraftübertragungsglied gekoppelt sein.

Vorteilhafterweise ist die Betätigungseinrichtung ausgebildet zum Bewegen der zwei Werkzeugelemente relativ zueinander. Dies ermöglicht eine besonders einfache Handhabung des Instruments, da ein Operateur durch Betätigen der Betätigungseinrichtung beispielsweise an einem distalen Ende eines Schafts relativ zueinander beweglich gelagerte Werkzeugelemente bewegen kann.

Günstig ist es insbesondere, wenn die Betätigungseinrichtung und das Kraftübertragungsglied beweglich miteinander gekoppelt sind zum Übertragen einer Betätigungskraft von der Betätigungseinrichtung auf das Kraftübertragungsglied. Diese Ausgestaltung gestattet es einem Operateur lediglich durch Handhaben der Betätigungseinrichtung das Kraftübertragungsglied zu bewegen und, wenn dieses mit einem oder beiden Werkzeugelementen gekoppelt ist, diese auch relativ zueinander in gewünschter Weise zu positionieren.

Die Handhabung des Instruments wird für einen Operateur besonders einfach, wenn die Betätigungseinrichtung am proximalen Ende des Instruments angeordnet oder ausgebildet ist. Er kann dann beispielsweise von außerhalb eines Patientenkörpers gezielt einen im Inneren des Patientenkörpers zu entfernenden Clip fassen, aufspreizen und insbesondere durch einen minimalinvasiven Zugang aus dem Patientenkörper entfernen.

Günstig ist es, wenn die zwei Werkzeugelemente in der Einführstellung und/oder der Clipentnahmestellung eine geschlossene Stellung einnehmen. Diese Ausgestaltung ermöglicht insbesondere das einfache Einführen und Herausziehen des Instruments aus einem Patientenkörper durch einen minimalinvasiven Zugang.

Vorteilhafterweise liegen die zwei Werkzeugelemente in der geschlossenen Stellung mindestens abschnittsweise aneinander an. Sie bilden damit gegenseitig Anschläge, um eine Bewegung aufeinander zu zu begrenzen. Damit kann auf einfache Weise auch die Clipaufnahme definiert werden, um sicherzustellen, dass zu entfernende Clips auch in definierter und gewünschter Weise gefasst und gehalten werden können.

Gemäß einer weiteren bevorzugten Ausführungsform der Erfindung kann eine Kulissenführung zum Definieren einer Verschwenkbewegung der zwei Werkzeugelemente relativ zueinander infolge einer Verschiebung des Kraftübertragungsglieds in Längsrichtung vorgesehen sein. Durch entsprechende Gestaltung der Kulissenführung ist es möglich, insbesondere eine definierte Bewegung zum Öffnen und Schließen der Werkzeugelemente, die auch als Maulteile bezeichnet werden können, sicherzustellen. Die Kulissenführung ermöglicht insbesondere eine Transformation einer Linearbewegung in eine Schwenkbewegung oder eine Bewegung senkrecht zur Längsachse.

Besonders einfach in seinem Aufbau wird das Instrument, wenn die Kulissenführung in Form einer Schlitz-Nocken-Führung ausgebildet ist, mit einem Führungsschlitz und einem in diesen geführt gehaltenen Nocken, wobei der Führungsschlitz am Schaft oder an einem Werkzeugelement und der korrespondierende Nocken am jeweils anderen Teil angeordnet oder ausgebildet ist. Durch eine Relativbewegung von Nocken und Führungsschlitz(en) kann so insbesondere eine Relativbewegung zwischen den Werkzeugelementen und gegebenenfalls auch relativ zum Schaft erreicht werden.

Günstig ist es, wenn jedes Werkzeugselement einen Führungsschlitz umfasst, und wenn ein am Schaft gehaltener Nocken in den beiden Führungsschlitzen geführt ist. So kann beispielsweise infolge einer Relativbewegung der Führungselemente in Längsrichtung des Schafts eine Bewegung quer zur Längsrichtung durch den in den Führungsschlitzen geführten Nocken erzwungen werden. Der Nocken kann insbesondere ausgebildet sein in Form eines die Führungsschlitze durchsetzenden oder in diese eingreifenden Stifts. Die Führungsschlitze können geradlinig und/oder gekrümmt ausgebildet sein.

Um einen Clip in der Clipaufnahme aufnehmen zu können, ist es vorteilhaft, wenn die Werkzeugelemente in der Clipaufnahmestellung eine geöffnete Stellung einnehmen.

Ferner ist es günstig, wenn die Werkzeugelemente in der Clipspreizstellung eine geöffnete Stellung einnehmen. Auf diese Weise kann insbesondere erreicht werden, dass freie Enden der Klemmarme oder in einer Anlegestellung aneinander anliegende Verbindungsbereiche des Clips in der Clipspreizstellung voneinander entfernt gehalten werden können.

Günstig kann es ferner sein, wenn die Werkzeugelemente relativ zum Schaft beim Übergang von der Clipaufnahmestellung in die Clipspreizstellung in proximaler Richtung verschoben werden. So kann insbesondere auf einfache Weise ein Abstand zwischen dem mindestens einen proximalen und dem mindestens einen distalen Clipaufnahmeanschlag verringert werden beim Übergang von der Clipaufnahmestellung in die Clipspreizstellung.

Des Weiteren kann es günstig sein, wenn die Schwenkachse, um die die Werkzeugelemente relativ zum Schaft verschwenkbar gelagert sind, beim Übergang von der Clipaufnahmestellung in die Clipspreizstellung in proximaler Richtung verschoben wird. Auf diese Weise ist es möglich, dass die Werkzeugelemente relativ zueinander und gegebenenfalls relativ zum Schaft eine überlagerte Translations-Schwenkbewegung ausführen.

Damit sichergestellt werden kann, dass der Clip, insbesondere dessen Verbindungsbereich, nicht so einfach aus der Clipaufnahme herausfallen kann, ist es günstig, wenn der proximale Clipaufnahmeanschlag in Form einer Ausnehmung ausgebildet ist, in welche der Verbindungsbereich des zu entfernenden Clips teilweise einführbar ist. Insbesondere können so Bewegungen quer zu einer von der Spreizeinrichtung definierten Längsrichtung gegebenenfalls auf einfache und sichere Weise verhindert werden.

Um den Clip auf einfache Weise aufnehmen zu können, ist es günstig, wenn die Ausnehmung in distaler Richtung weisend geöffnet ist. So kann die Spreizeinrichtung mit dem mindestens einen proximalen Clipaufnahmeanschlag durch eine Bewegung in distaler Richtung insbesondere an den Verbindungsbereich eines einfachen Clips oder an freie Enden eines Doppelstegclips herangeführt werden.

Gemäß einer weiteren bevorzugten Ausführungsform der Erfindung kann vorgesehen sein, dass der distale Clipaufnahmeanschlag in Form einer Klemmbackenausnehmung ausgebildet ist, in welche insbesondere ein Verbindungsbereich des zu entfernenden Clips teilweise einführbar ist. So kann insbesondere eine Bewegung des Clips, insbesondere seiner freien Enden der Klemmarme oder seiner Verbindungsbereiche relativ zur Spreizeinrichtung einfach und sicher verhindert werden, so dass der Clip definiert und sicher in der Clipaufnahme haltbar ist. Insbesondere kann der Clip so in der Ausnehmung einerseits und in der Klemmbackenausnehmung beziehungsweise in den Klemmbackenausnehmungen andererseits definiert gehalten werden.

Vorzugsweise ist die Klemmbackenausnehmung in proximaler Richtung weisend geöffnet. Sie kann somit insbesondere in Richtung auf die Ausnehmung hin weisen, die den mindestens einen proximalen Clipaufnahmeanschlag definiert. So kann ein Clip, auch ohne dass er Hinterschnitte oder abstehende Vorsprünge aufweist, an denen er alternativ gehalten werden könnte, einerseits an seinem oder seinen Verbindungsbereich(en) und andererseits im Bereich freier Ende seiner Klemmarme sicher gefasst und gehalten werden.

Günstig ist es, wenn die Spreizeinrichtung ein in proximaler Richtung weisendes Spreizglied umfasst, welches insbesondere zwischen zwei Verbindungsbereiche der Klemmarme des zu entfernenden Clips einführbar ist. Das Spreizglied kann so insbesondere in Richtung auf den mindestens einen proximalen Clipaufnahmeanschlag hin weisen. Insbesondere kann das Spreizglied relativ zum mindestens einen proximalen Clipaufnahmeanschlag bewegbar ausgebildet sein, vorzugsweise in Richtung auf diesen hin, um infolge einer derartigen Bewegung die Klemmarme des Clips aufzuspreizen.

Besonders einfach und sicher aufspreizen lässt sich ein chirurgischer Clip, wenn das Spreizglied keilförmig ausgebildet ist und eine in proximaler Richtung weisende Spreizkante umfasst. So kann das Spreizglied beispielsweise zwischen zwei aneinander anliegende Klemmarme ausgehend von deren freien Enden oder zwischen zwei aneinander anliegende Verbindungsbereiche eingeschoben werden, wobei die freien Enden beziehungsweise die Verbindungsbereiche dann am keilförmigen Spreizglied aufgleiten und aufgespreizt werden können.

Besonders kompakt ausbilden lässt sich die Spreizeinrichtung, wenn die zwei Werkzeugelemente jeweils ein Spreizgliedteil umfassen und wenn die Spreizgliedteile das Spreizglied bilden. Mit anderen Worten ist das Spreizglied somit zweiteilig ausgebildet beziehungsweise mindestens zweiteilig ausgebildet. Insbesondere können die beiden Spreizgliedteile jeweils einen distalen Clipaufnahmeanschlag umfassen oder an einen solchen angrenzend ausgebildet sein.

Damit die Spreizeinrichtung in der Einführstellung einen möglichst kompakten Aufbau aufweist, ist es günstig, wenn die Spreizgliedteile in der Einführ- und/oder der Clipentnahmestellung aneinander anliegen zur Ausbildung des Spreizglieds. Insbesondere ist es so möglich, einen möglichst kleinen Querschnitt der Spreizeinrichtung zu definieren, um das Instrument durch einen minimalinvasiven Zugang in einen Patientenkörper einführen und wieder aus diesem herausziehen zu können.

Zur Verbesserung der Taktilität des Instruments ist es günstig, wenn dieses eine erste Anschlageinrichtung zum Definieren der Einführstellung aufweist. Die erste Anschlageinrichtung kann insbesondere derart ausgebildet sein, dass ein Operateur eine für ihn spürbare Rückmeldung bekommt, wenn das Instrument die Einführstellung einnimmt. Er kann dann, auch ohne Sichtkontakt zur Spreizeinrichtung, sicherstellen, dass beispielsweise die beiden Werkzeugelemente eine geschlossene oder Schließstellung einnehmen.

Damit das Instrument noch eine weiter verbesserte Taktilität aufweist, ist es günstig, wenn es eine zweite Anschlageinrichtung zum Definieren der Clipentnahmestellung umfasst. Nach Aufnehmen und Aufspreizen des Clips ist es so für einen Operateur möglich, das Instrument in die Clipentnahmestellung zu überführen, ohne die Spreizeinrichtung sehen zu müssen. Durch die zweite Anschlageinrichtung kann je nach Konstruktion insbesondere auch sichergestellt werden, dass das Instrument in keine andere Stellung mehr gebracht werden kann, wenn es die Clipentnahmestellung einmal eingenommen hat. Dadurch kann insbesondere sichergestellt werden, dass ein in der Clipaufnahme gehaltener Clip, der nach Aufnehmen und Aufspreizen in dieser beispielsweise nach Entfernen vom Gefäß oder Hohlorgan wieder geschlossen ist, nicht unbeabsichtigt aus der Clipaufnahme herausfallen kann.

Günstig ist es, wenn die erste und/oder zweite Anschlageinrichtung an der Betätigungseinrichtung angeordnet oder ausgebildet sind. Dies hat insbesondere den Vorteil, dass ein sehr kompakter Aufbau der Spreizeinrichtung möglich ist. Zudem kann die Anschlageinrichtung an der Betätigungseinrichtung insbesondere derart ausgebildet werden, dass das Erreichen der Clipentnahmestellung für einen Operateur nicht nur spürbar, sondern auch sichtbar wird. Alternativ ist es selbstverständlich auch denkbar, die erste und/oder die zweite Anschlageinrichtung am Schaft oder insbesondere auch an der oder im Bereich der Spreizeinrichtung anzuordnen oder auszubilden.

Vorzugsweise sind die erste und/oder zweite Anschlageinrichtung ausgebildet zum Begrenzen einer Bewegung relativ zueinander bewegbarer Betätigungsglieder der Betätigungseinrichtung. Insbesondere bei einer Betätigungseinrichtung, die zwei oder gegebenenfalls auch mehr Betätigungsglieder aufweist, kann durch eine einfache Begrenzung der Bewegung derselben relativ zueinander auch beispielsweise die Einführstellung und/oder die Clipentnahmestellung definiert werden. Die Betätigungsglieder können insbesondere mit dem Schaft einerseits und mit einem oder beiden Werkzeugelementen gekoppelt sein, um infolge einer Bewegung der Betätigungsglieder relativ zueinander auch die Werkzeugelemente und damit die Clipaufnahmeanschläge relativ zueinander bewegen zu können.

Günstig ist es, wenn die Betätigungsglieder in einer maximal weit voneinander entfernten Stellung die Clipaufnahmestellung definieren. Durch voneinander weg Bewegen der Betätigungsglieder kann ein Operateur so auf einfache Weise das Instrument in die Clipaufnahmestellung überführen.

Ferner kann es vorteilhaft sein, wenn die Betätigungsglieder in einer maximal angenäherten Stellung die Clipspreizstellung definieren. Auf diese Weise wird insbesondere die Handhabbarkeit des Instruments verbessert, da ein Operateur die Betätigungsglieder einfach gegeneinander bewegen muss, bis diese nicht mehr weiter aufeinander zu bewegt werden können. Dann kann er sicher sein, dass der Clip maximal weit aufgespreizt ist und auf einfache Weise vom Gefäß oder Hohlorgan entfernt werden kann.

Gemäß einer weiteren bevorzugten Ausführungsform der Erfindung kann vorgesehen sein, dass die erste Anschlageinrichtung derart angeordnet oder ausgebildet ist, dass die Betätigungsglieder beim Übergang von der maximal weit voneinander entfernten Stellung in die maximal angenäherte Stellung eine definierte Zwischenstellung einnehmen, in welcher die Werkzeugelemente geschlossen sind. So ist es auf einfache Weise möglich, die erste Anschlageinrichtung so zu gestalten, dass das Instrument die Einführstellung einnimmt. Insbesondere kann an der definierten Zwischenstellung der Betätigungsglieder ein Operateur erkennen, dass das Instrument die Einführstellung einnimmt, auch wenn er nicht die Spreizeinrichtung direkt sehen kann.

Besonders einfach wird der Aufbau des Instruments, wenn die erste Anschlageinrichtung einen Zwischenanschlag umfasst, welcher an einem der Betätigungsglieder angeordnet ist und mit dem anderen Betätigungsglied zum Definieren der Einführstellung zusammenwirkt. Der Zwischenanschlag kann insbesondere feststehend oder alternativ auch bewegbar an dem einen Betätigungsglied angeordnet sein. Beispielsweise kann er so ausgebildet sein, dass er entgegen der Wirkung eines Rückstellglieds, zum Beispiel einer Druckfeder, mit einer definierten Rückstellkraft bewegt werden kann, um insbesondere das Instrument von der Einführstellung in die Clipspreizstellung zu überführen. Alternativ wäre es auch möglich, den Zwischenanschlag verformbar auszubilden, wobei eine bestimmte Kraft zu dessen Verformung erforderlich sein muss.

Vorteilhafterweise ist der Zwischenanschlag zum Übergang von der Einführstellung in die Clipspreizstellung temporär deaktivierbar. Wie bereits im vorstehenden Absatz dargelegt, kann der Zwischenanschlag beispielsweise deaktivierbar oder ausschaltbar sein, indem er relativ zu dem Betätigungsglied, an dem er angeordnet oder ausgebildet ist, bewegt werden kann, um eine Bewegung der Betätigungsglieder und damit auch des Instruments von der Einführstellung in die Clipspreizstellung zu ermöglichen. Insbesondere kann der Anschlag ausgebildet sein mit einer Aufgleitfläche an welcher ein entsprechender Vorsprung des anderen Betätigungsglieds aufgleiten und den Zwischenanschlag entgegen der Wirkung eines Rückstellglieds verschieben kann. Die Wirkung des Rückstellglieds ist für einen Operateur spürbar, so dass er bei Erhöhung einer Kraft zum Bewegen der Betätigungsglieder, beispielsweise aufeinander zu, erspüren kann, wenn das Instrument die Einführstellung einnimmt.

Vorteilhaft ist es, wenn der Zwischenanschlag eine in distaler Richtung weisende Zwischenanschlagfläche umfasst. So kann auf einfache Weise ein definierter Anschlag ausgebildet werden. Insbesondere kann die Zwischenanschlagfläche in der beschriebenen Weise als Aufgleitfläche ausgebildet sein, um beim Aufgleiten eines Teils des anderen Betätigungsglieds den Zwischenanschlag zu bewegen, zum Beispiel um die Einführstellung gegebenenfalls freizugeben.

Gemäß einer weiteren bevorzugten Ausführungsform der Erfindung kann es vorteilhaft sein, wenn die zweite Anschlageinrichtung einen Endanschlag umfasst, welcher an einem der Betätigungsglieder angeordnet ist und mit dem anderen Betätigungsglied zum Definieren der Clipentnahmestellung zusammenwirkt. Insbesondere kann der Endanschlag derart ausgebildet sein, dass das Instrument nicht mehr in die Clipspreiz- oder Clipaufnahmestellung überführt werden kann. So kann insbesondere sichergestellt werden, dass ein von einem Gefäß oder Hohlorgan entfernter Clip beim Herausziehen des Instruments aus einem Patientenkörper sicher in der Clipaufnahme gehalten werden kann.

Vorteilhafterweise ist der Endanschlag zum Übergang von der Clipentnahmestellung in die Einführstellung temporär deaktivierbar. Dies ist insbesondere von Vorteil, wenn das Instrument in Form eines wiederverwendbaren Instruments ausgebildet ist. Das beispielsweise durch die zweite Anschlageinrichtung mit ihrem Endanschlag dauerhaft blockierte Instrument kann beispielsweise so ausgebildet sein, dass es nur durch entsprechenden manuellen Eingriff wieder in die Einführstellung überführbar ist. Auf diese Weise kann beispielsweise sichergestellt werden, dass erst nach sicherer Entnahme des Clips aus dem Patientenkörper und Entnahme des Clips aus der Clipaufnahme das Instrument wieder zur Entnahme eines weiteren Clips bereit gemacht werden kann. Beispielsweise kann eine temporäre Deaktivierung derart erfolgen, dass eine Bedienperson manuell gegen die Zwischenanschlagfläche drückt, welche in ihrer Position durch ein Rückstellglied vorgespannt sein kann.

Vorteilhaft ist es, wenn der Endanschlag eine in proximaler Richtung weisende Endanschlagfläche umfasst. So kann auf einfache und definierte Weise eine Bewegung der Betätigungsglieder beispielsweise voneinander weg oder alternativ auch aufeinander zu verhindert werden.

Besonders einfach wird der Aufbau des Instruments, wenn die erste und die zweite Anschlageinrichtung einen Vorsprung umfassen, welcher die Zwischenanschlagfläche und/oder die Endanschlagfläche umfasst. Der Vorsprung kann so insbesondere sowohl den Zwischenanschlag als auch den Endanschlag bilden, so dass nur eine minimale Anzahl an Teilen zur Ausbildung des Instruments erforderlich ist.

Um zu verhindern, dass ein von einem Gefäß oder Hohlorgan entfernter Clip aus der Clipaufnahme herausfallen kann, ist es vorteilhaft, wenn das Instrument eine Sicherungseinrichtung zum Sichern desselben in der Clipentnahmestellung umfasst. Die Sicherungseinrichtung kann es insbesondere ermöglichen, das Instrument so auszugestalten, dass es, sobald es die Clipentnahmestellung erreicht hat, in keine andere Stellung mehr überführt werden kann.

Auf diese Weise kann insbesondere verhindert werden, dass ein in der Clipaufnahme aufgenommener Clip aus dieser unbeabsichtigt herausfallen kann.

Ein besonders kompakter Aufbau des Instruments kann insbesondere dadurch erreicht werden, dass die Sicherungseinrichtung die zweite Anschlageinrichtung umfasst.

Die Handhabbarkeit des Instruments kann insbesondere dadurch weiter verbessert werden, dass die Betätigungseinrichtung und der Schaft relativ zueinander um eine Längsachse des Schafts verdrehbar sind. Dies ermöglicht es einem Operateur, den Schaft und den Handgriff relativ zueinander so zu bewegen, dass er in einer für ihn ergonomischen Stellung der Betätigungseinrichtung die Spreizeinrichtung derart orientieren kann, damit ein Clip einfach und sicher aufgenommen werden kann.

Um die Reinigbarkeit zu verbessern und zudem das Instrument für die Entnahme mehrerer Clips wieder schneller verfügbar zu machen, ist es vorteilhaft, wenn das Instrument einen mit dem Schaft lösbar verbindbaren Instrumentengriff umfasst, welcher die Betätigungseinrichtung umfasst. Beispielsweise können so der Instrumentengriff und der Schaft getrennt voneinander gereinigt werden. Zudem kann optional der Schaft vom Instrumentengriff gelöst werden, wenn ein Clip mit dem Instrument entnommen wurde. Der Instrumentengriff kann dann mit einem weiteren Schaft verbunden werden, der bereits wieder zur Aufnahme eines Clips bereit ist, während der Schaft, mit dem gerade ein Clip entnommen wurde, zur Aufnahme eines weiteren Clips bereit gemacht werden kann, insbesondere durch Herausnehmen des Clips aus der Clipaufnahme.

Die nachfolgende Beschreibung bevorzugter Ausführungsformen der Erfindung dient im Zusammenhang mit der Zeichnung der näheren Erläuterung. Es zeigen:
- Figur 1:: eine schematische, teilweise durchbrochene Seitenansicht eines chirurgischen Instruments zum Entfernen eines an ein Hohlorgan angelegten chirurgischen Clips;
- Figur 2:: eine Explosionsdarstellung einer Spreizeinrichtung des Instruments aus Figur 1;
- Figur 3:: eine teilweise geschnittene Längsschnittansicht der Spreizeinrichtung in der Einführstellung;
- Figur 4:: eine teilweise geschnittene Längsschnittansicht des Instrumentengriffs in der Einführstellung;
- Figur 5:: eine Ansicht der Spreizeinrichtung analog Figur 3 in der Clipaufnahmestellung;
- Figur 6:: eine Ansicht des Instrumentengriffs analog Figur 4 in der Clipaufnahmestellung;
- Figur 7:: eine Ansicht der Spreizeinrichtung analog Figur 3 in der Clipaufspreizstellung;
- Figur 8:: eine Ansicht des Instrumentengriffs analog Figur 4 in der Clipaufspreizstellung;
- Figur 9:: eine Ansicht der Spreizeinrichtung analog Figur 3 in der Clipentnahmestellung;
- Figur 10:: eine Ansicht des Instrumentengriffs analog Figur 4 in der Clipentnahmestellung;
- Figur 11:: eine schematische Darstellung der Vorgehensweise beim Entfernen eines chirurgischen Clips;
- Figur 12:: eine schematische Darstellung einer alternativen Variante zum Entfernen eines Clips;
- Figur 13:: eine schematische Darstellung eines geschlossenen Clips vor dem nicht erfindungsgemäßen Entfernen durch Aufquetschen; und
- Figur 14:: eine schematische Darstellung des nicht erfindungsgemäß aufgequetschten Clips.

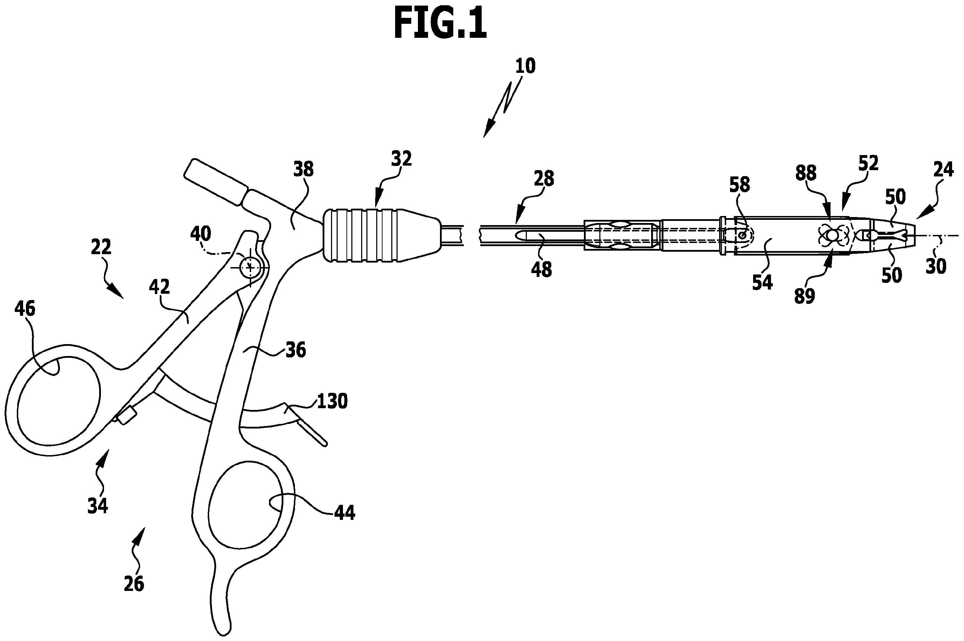

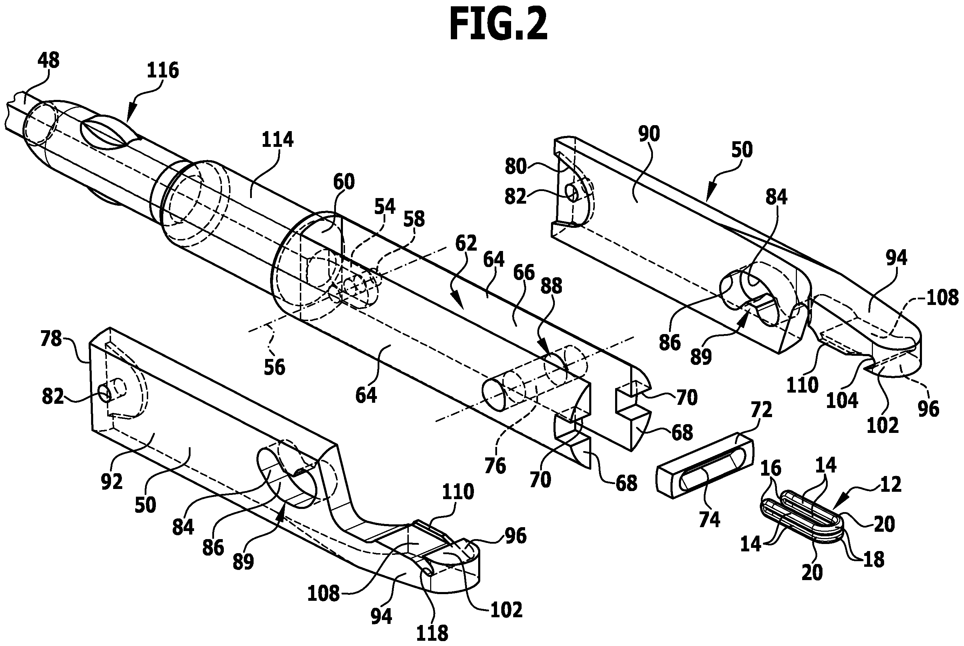

In Figur 1 ist ein insgesamt mit dem Bezugszeichen 10 bezeichnetes chirurgisches Instrument zum Entfernen eines an ein Hohlorgan angelegten chirurgischen Clips 12 schematisch dargestellt. Der Clip 12 kann insbesondere in Form eines in Figur 2 dargestellten Doppelstegclips ausgebildet sein, welcher vier jeweils paarweise aneinander anliegende Klemmarme 14 aufweist, die vier freie, jeweils paarweise verbundene Enden 16 und vier ebenfalls paarweise miteinander verbundene und jeweils einen Verbindungsbereich 18 definierende Enden 20 aufweisen. Das Instrument 10 weist ein proximales Ende 22 sowie ein distales Ende 24 auf.

Das Instrument 10 umfasst einen Instrumentengriff 26, welcher mit einem langgestreckten rohrförmigen Schaft 28 lösbar verbindbar ist. Der Instrumentengriff 26 und der Schaft 28 sind relativ zueinander um eine vom Schaft 28 definierte Längsachse 30 rotierbar. Eine Verbindung zwischen Instrumentengriff 26 und Schaft 28 wird hergestellt durch ein am Instrumentengriff 26 bewegbar gehaltenes Kupplungsstück 32.

Der Instrumentengriff 26 umfasst eine Betätigungseinrichtung 34, welche ein erstes Betätigungsglied 36 in Form einer mit einem Instrumentengriffgrundkörper 38 fest verbundenen und im Wesentlichen quer zur Längsachse 30 abstehenden Branche sowie ein am Instrumentengriffgrundkörper 38 um eine senkrecht zur Längsachse 30 verlaufende Schwenkachse 40 verschwenkbares zweites, branchenförmiges Betätigungsglied 42 umfasst, das etwas weiter in proximaler Richtung abstehend gelagert ist. Freie Enden der ersten und zweiten Betätigungsglieder 36, 42 sind in Form von Fingerringen 44 beziehungsweise 46 ausgebildet.

Die Betätigungseinrichtung 34 dient zum Übertragen einer Betätigungskraft über ein beweglich mit dem zweiten Betätigungsglied 42 gekoppeltes Kraftübertragungsglied 48 in Form einer im Schaft 28 verschiebbar und rotierbar gelagerten Schub- und Zugstange auf zwei am distalen Ende des Schafts beweglich gelagerte, identisch ausgebildete, jedoch relativ zueinander um 180° verdreht um die Längsachse 30 angeordnete Werkzeugelemente 50 einer Spreizeinrichtung 52 zum Fassen und Aufspreizen eines angelegten Clips 12 von einer Anlegestellung in eine Lösestellung. Ein distales Ende des Kraftübertragungsglieds 48 ist in Form eines Lagerbackens 54 ausgebildet, der eine Querbohrung aufweist, in der ein koaxial zu einer senkrecht zur Längsachse 30 verlaufenden Schwenkachse 56 eingesetzter Lagerstift 58 gehalten ist.

Der Lagerbacken 54 steht in distaler Richtung weisend über einen Boden 60 einer im Wesentlichen U-förmigen Werkzeugelementaufnahme 62 vor, die spiegelsymmetrisch zu einer die Längsachse 30 enthaltenden Spiegelebene ausgebildet ist und zwei einander gegenüberliegende seitliche Schenkel 64 umfasst, die zueinander parallele Innenwände 66 aufweisen. In in distaler Richtung weisenden Endflächen 68 der Schenkel 64 ist jeweils eine Quernut 70 ausgebildet, in die ein im Wesentlichen quaderförmiger proximaler Clipaufnahmeanschlag 72 eingesetzt ist, welcher eine in distaler Richtung weisende Ausnehmung 74 aufweist. Etwas proximalseitig der Quernut 70 ist in Querbohrungen der Schenkel 64 ein rundstabförmiger Nocken 76 eingesetzt.

Die Werkzeugelemente 50 weisen ausgehend von ihrem proximalen Ende 78 eine in proximaler Richtung und in Richtung auf die Längsachse 30 hin weisende, im Wesentlichen halbkreisförmige Ausnehmung 80 auf, die zusätzlich mit einer Querbohrung 82 versehen ist. Die beiden Werkzeugelemente weisen ferner etwas distalseitig der Mitte sich quer zur Längsachse 30 erstreckende, im Wesentlichen halbkreisförmig gekrümmte Durchbrechungen 84 auf, die Führungsschlitze 86 einer Kulissenführung 89 in Form einer insgesamt mit dem Bezugszeichen 88 versehenen Schlitz-Nocken-Führung bilden. Die beiden Werkzeugelemente 50 liegen mit Innenflächen 90 aneinander an und mit parallel zu diesen verlaufenden Außenflächen 92 an den Innenwänden 66 der Schenkel 64. Der beidseitig über den Lagerbacken 54 vorstehende Lagerstift 58 greift in die Querbohrungen 82 ein und koppelt so die Werkzeugelemente 50 beweglich mit dem Kraftübertragungsglied 48. Der Nocken 76 ist in den Führungsschlitzen 86 geführt, so dass es infolge einer Bewegung des Kraftübertragungsglieds 48 in proximaler beziehungsweise distaler Richtung zu einer zwangsgeführten Schwenkbewegung der Werkzeugelemente 50 um die gemeinsame Schwenkachse 56 kommt. Diese wird wiederum infolge einer Bewegung des Kraftübertragungsglieds 48 in proximaler Richtung ebenfalls in proximaler Richtung bewegt. Eine Bewegung des Kraftübertragungsglieds 48 in distaler Richtung führt zwangsläufig zu einer Bewegung des Lagerstifts 58 und damit auch der Schwenkachse 56 in distaler Richtung.

Distale Endabschnitte 94 der Werkzeugelemente 50 definieren in einer geschlossenen Stellung, in welcher aufeinander zu weisende Anlageflächen 96 aneinander anliegen, ein eine in proximaler Richtung weisende Spreizkante 98 umfassendes Spreizglied 100. Das Spreizglied 100 ist zweiteilig ausgebildet, und zwar durch zwei Spreizgliedteile 102, die jeweils an einem der beiden Werkzeugelemente 50 ausgebildet sind. Die Spreizgliedteile 102 werden insbesondere seitlich begrenzt durch die Anlageflächen 96. Ausgehend von der Spreizkante 98 definiert jedes Werkzeugelement 50 eine in proximaler Richtung weisende Klemmbackenausnehmung 104, die in distaler Richtung voneinander weg geöffnete Mittelebenen 106 definieren. Etwas proximalseitig der Spreizkante 98 weisen die Werkzeugelemente 50 aufeinander zu weisende, jedoch voneinander beabstandete Seitenflächen 108 auf, die jeweils auf einer Seite einen parallel zur Längsachse 30 verlaufenden Rand 110 aufweisen. Eine Höhe des Rands 110 entspricht etwa dem halben Abstand zwischen den Seitenflächen 108, wenn die Anlageflächen 96 aneinander anliegen. Durch die Seitenflächen 108 sowie die Ausnehmung 74 und die Klemmbackenausnehmung 104 wird eine Clipaufnahme 112 der Spreizeinrichtung 52 definiert. Die Ränder 110 schließen die Clipaufnahme 112 seitlich zumindest teilweise.

Proximalseitig der Schenkel 64 ist eine Führungshülse 114 koaxial zur Längsachse 30 in proximaler Richtung abstehend angeordnet, die eine vom Kraftübertragungsglied 48 durchsetzte Längsbohrung umfasst. Proximalseitig schließt sich an die Führungshülse 114 ein Kupplungsabschnitt 116 an, mit dem die Spreizeinrichtung 52 mit dem Schaft 28 optional lösbar verbindbar ist.

Der Clip 12, wie er beispielsweise in Figur 2 dargestellt ist, ist in Form eines Doppelstegclips ausgebildet, wobei die freien Enden 16 der Klemmarme 14 mit freien Enden 16 zweier weiterer Klemmarme 14 verbunden sind, deren gegenüberliegende freie Enden wiederum über einen Verbindungsbereich 18 miteinander verbunden sind. In einer Draufsicht ist der Clip 12 im Wesentlichen U-förmig ausgebildet, wobei die Klemmarme 14, die über den Verbindungsbereich 18 miteinander verbunden sind, parallel zueinander verlaufen und voneinander beabstandet sind. Die über ihre freien Enden 16 miteinander verbundenen Klemmarme 14 liegen in einer Anlegestellung des Clips 12 flächig aneinander an, ebenso wie die Verbindungsbereiche 18. Der Clip 12 ist aus einem in sich geschlossenen, im Wesentlichen drahtförmig gebildeten Material ausgebildet. In einer Draufsicht ist der Clip 12 in Richtung auf die freien Enden 16 hin geöffnet. In einer Seitenansicht ist der Clip 12 in Richtung auf die Verbindungsbereiche 18 hin weisend geöffnet, wobei, wie bereits erwähnt, in der Seitenansicht die über die freien Enden 16 verbundenen Klemmarme 14 flächig aneinander anliegen.

Die Clipaufnahme 12 ist derart ausgebildet, dass die freien Enden 16 in die Ausnehmung 74 und die Verbindungsbereiche 18 jeweils in eine der durch die Klemmbackenausnehmung 104 definierten distalen Clipaufnahmeanschläge 118 eingreifen können. Die proximalen und distalen Clipaufnahmeanschläge 72 und 118 begrenzen die Clipaufnahme 112 proximalseitig beziehungsweise distalseitig. Die proximalen und distalen Clipaufnahmeanschlage 72, 118 definieren zwischen sich einen Abstand 120. Die beiden distalen Clipaufnahmeanschläge 118 definieren zwischen sich einen Querabstand 122.

Das Instrument 10 umfasst ferner eine insgesamt mit dem Bezugszeichen 124 versehene erste Anschlageinrichtung sowie eine insgesamt mit dem Bezugszeichen 126 versehene zweite Anschlageinrichtung. Beide Anschlageinrichtungen 124 und 126 sind an der Betätigungseinrichtung 34 angeordnet beziehungsweise ausgebildet. Sie dienen zur Begrenzung einer Bewegung der Betätigungsglieder 36 und 42 relativ zueinander.

Die erste Anschlageinrichtung 124 umfasst einen Zwischenanschlag 128, welcher an einem koaxial bezogen auf die Schwenkachse 40 gekrümmten Hebelarm 130 in Richtung auf die Schwenkachse 40 hin abstehend angeordnet ist. Der Hebelarm 130 ist vom zweiten Betätigungsglied 42 in Richtung auf das erste Betätigungsglied 36 abstehend angeordnet und durchsetzt eine in letzterem ausgebildete Durchbrechung 132. Der Zwischenanschlag 128 wirkt somit mit dem ersten Betätigungsglied 36 zusammen, und zwar mit einer in proximaler Richtung weisenden Seitenfläche 134 desselben, die bis an die Durchbrechung 132 heranreicht. Der Zwischenanschlag 128 umfasst eine im Wesentlichen in distaler Richtung weisende Zwischenanschlagfläche 136, die etwas in Richtung auf die Längsachse 30 hin geneigt ist. Sie bildet eine Aufgleitfläche für die Seitenfläche 134. Vorzugsweise ist der Zwischenanschlag 128 in nicht dargestellter Weise entgegen der Wirkung eines Rückstellglieds, beispielsweise einer Schraubenfeder, in Richtung auf den Hebelarm 130 hin und in diesen hinein verschiebbar, wenn die Seitenfläche 134 an der Zwischenanschlagfläche 136 aufgleitet. Der Zwischenanschlag 128 definiert so einen von einer Bedienperson spürbaren Druckpunkt.

Die zweite Anschlageinrichtung 126 umfasst einen Endanschlag 138, welcher durch einen Vorsprung 140 definiert wird, der auch den Zwischenanschlag 128 bildet. Der Endanschlag 138 umfasst eine in proximaler Richtung weisende Endanschlagfläche 142, die im Wesentlichen senkrecht zum Hebelarm 130 abstehend ausgebildet ist. Sie wirkt mit einer in distaler Richtung weisenden Seitenfläche 144 des ersten Betätigungsglieds 36 zusammen.

Der Endanschlag 138 ist temporär deaktivierbar. Hierfür muss er manuell in Richtung auf den Hebelarm 130 hin und in diesen hinein bewegt werden, beispielsweise entgegen der Wirkung des beschriebenen Rückstellglieds. Eine temporäre Deaktivierung des Zwischenanschlags 128 erfolgt automatisch beim Bewegen des zweiten Betätigungsglieds 42 in Richtung auf das erste Betätigungsglied 36 hin, wenn die Seitenfläche 134 an der Zwischenanschlagfläche 136 aufgleitet und den Zwischenanschlag 128 entgegen der Wirkung des Rückstellglieds in Richtung auf den Hebelarm 130 hin bewegt.

Durch die besondere Ausgestaltung der zweiten Anschlageinrichtung 126 umfasst diese auch eine Sicherungseinrichtung 146, welche verhindert, dass das zweite Betätigungsglied 42 weiter in Richtung auf das erste Betätigungsglied 36 hin bewegt werden kann, wenn die Seitenfläche 144 an der Endanschlagfläche 142 anschlägt.

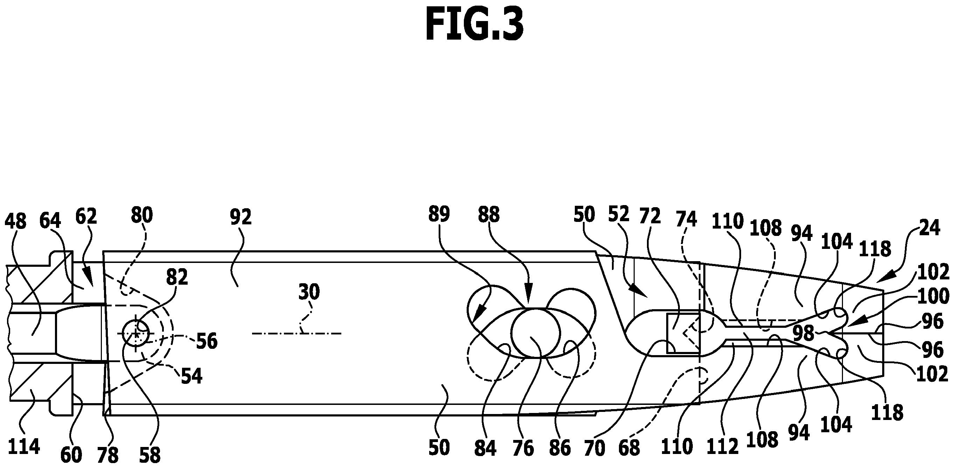

Die Funktionsweise des Instruments wird nachfolgend insbesondere in Verbindung mit den Figuren 3 bis 10 näher erläutert.

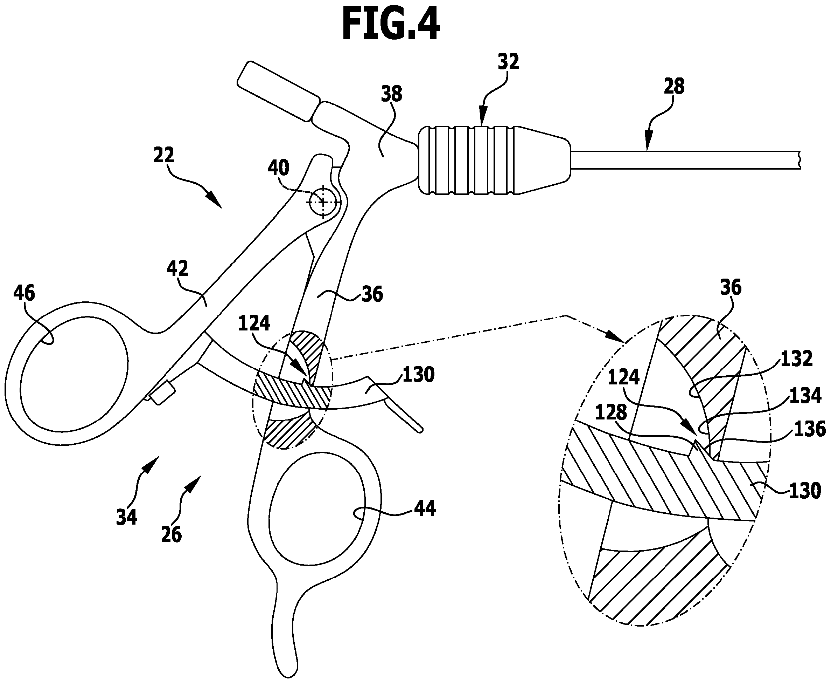

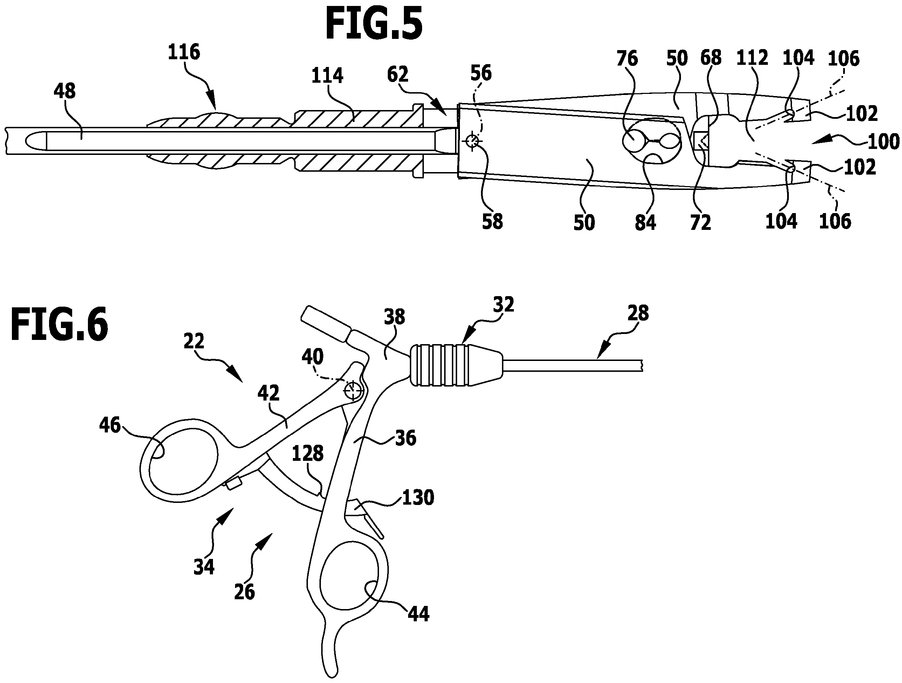

Zum Entfernen eines an ein Gefäß oder Hohlorgan angelegten Clips 12 muss das Instrument 10 mit seiner Spreizeinrichtung 52 zunächst, beispielsweise durch einen minimalinvasiven Zugang, in einen Patientenkörper hinein und an den zu entfernenden Clip 12 herangeführt werden. Dazu wird das Instrument 10 in die schematisch in den Figuren 3 und 4 dargestellte Einführstellung überführt. Hierzu wird ausgehend von einer maximal weit geöffneten Stellung der Werkzeugelemente 50, wie sie in Figur 5 dargestellt ist, das zweite Betätigungsglied 42 so weit in Richtung auf das erste Betätigungsglied 36 hin bewegt, bis die Seitenfläche 134 an der Zwischenanschlagfläche 136 anschlägt. Dies ist für einen Operateur als der oben beschriebene Druckpunkt spürbar. Die Anlageflächen 96 liegen in dieser Einführstellung flächig aneinander an. Der Nocken 76 nimmt eine Zwischenstellung in den beiden Führungsschlitzen 86 ein, wie in Figur 3 schematisch dargestellt.

Die Spreizeinrichtung 52 ist von der Einführstellung, in welcher die Clipaufnahme 112 eine minimale Querschnittsfläche definiert, in die schematisch in den Figuren 5 und 6 dargestellte Clipaufnahmestellung überführbar, in welcher die Clipaufnahme 112 zur Aufnahme des zu entfernenden Clips 12 geöffnet ist. Hierzu wird das zweite Betätigungsglied 42 vom ersten Betätigungsglied 36 weg verschwenkt. Die Betätigungsglieder 36 und 42 sind in der Clipaufnahmestellung maximal weit voneinander entfernt. Die Spreizeinrichtung 52 wird dann mit dem proximalen Clipaufnahmeanschlag 72 so an den Clip 12 herangeführt, dass die freien Enden 16, wie schematisch in der Explosionsdarstellung in Figur 2 gut zu erkennen, in die Ausnehmung 74 eingreifen können.

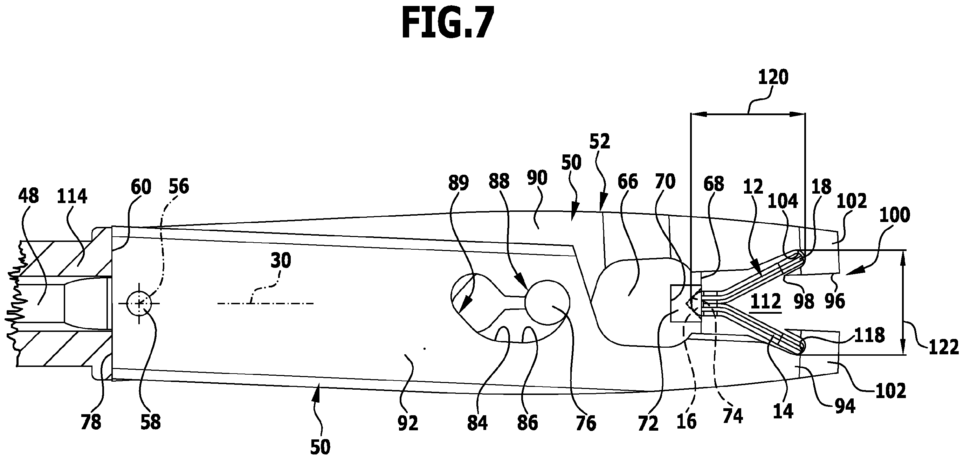

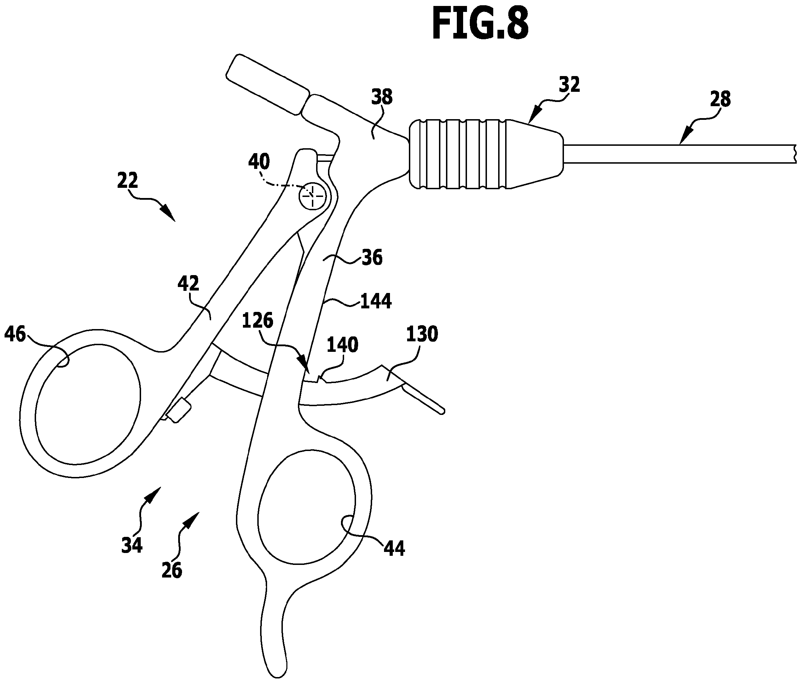

Im nächsten Schritt wird das Instrument 10 von der Clipaufnahmestellung in die Clipspreizstellung überführt, die schematisch in den Figuren 7 und 8 dargestellt ist. Hierzu wird nach Heranführen der freien Enden 16 an die Ausnehmung 74 das zweite Betätigungsglied 42 in Richtung auf das erste Betätigungsglied 36 hin bewegt, und zwar über den beschriebenen Druckpunkt, der durch die erste Anschlageinrichtung 124 definiert wird, hinweg bis zu einer maximal angenäherten Stellung der Betätigungsglieder 36 und 42. Infolge der Bewegung der Betätigungsglieder 36 und 42 aufeinander zu wird auch das Kraftübertragungsglied 48 in proximaler Richtung bewegt und der Nocken 76 wandert von einer proximalen Endposition in den Führungsschlitzen 86 bis zu einer distalen Endposition, wie sie schematisch in Figur 7 dargestellt ist.

In der Clipspreizstellung sind die Werkzeugelemente 50 nicht ganz so weit voneinander entfernt aufgespreizt wie in der Clipaufnahmestellung. Beim Übergang von der Clipaufnahmestellung in die Clipspreizstellung durchläuft die Spreizeinrichtung 52 die Einführstellung. Beim Übergang von der Clipaufnahmestellung in die Clipspreizstellung bewegen sich die distalen Clipaufnahmeanschläge 118 in Richtung auf den proximalen Clipaufnahmeanschlag 72 hin, so dass der Abstand 120 immer kleiner wird. Die Verbindungsbereiche 18, die jeweils in eine Klemmbackenausnehmung 104 eingreifen, werden dabei gezwungenermaßen voneinander weg bewegt, da der Querabstand 122 ausgehend von der Einführstellung in die Clipspreizstellung sukzessive größer wird. Das Aufspreizen wird insbesondere erleichtert durch das Spreizglied 100, das zwischen die Verbindungsbereiche 18 eingreift und diese in die Klemmbackenausnehmungen 104 hinein führt.

Der in der Clipaufnahme 112, wie in Figur 7 dargestellt, aufgespreizt gehaltene Clip 12 kann nun vom Gefäß oder Hohlorgan abgezogen werden, ohne dieses zu beschädigen.

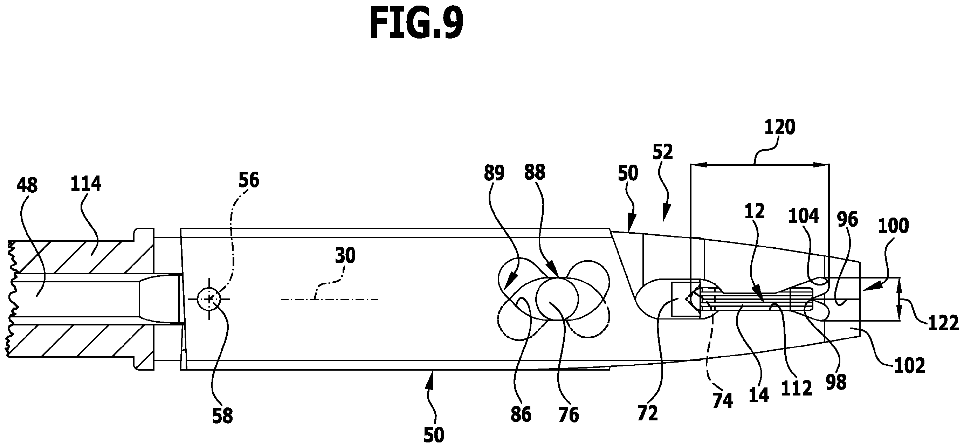

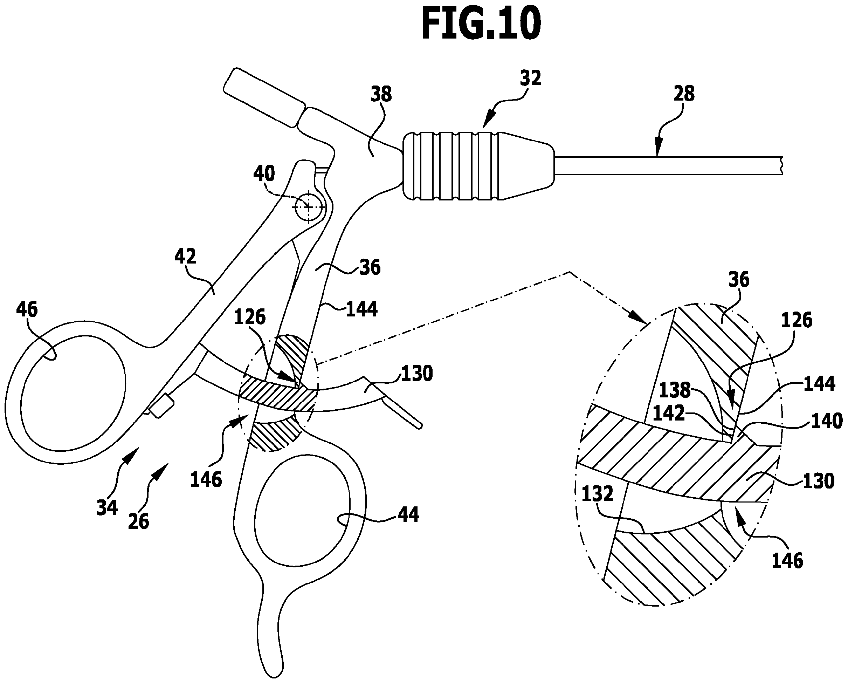

Da die aufgespreizten Werkzeugelemente 50 insbesondere bei einem minimalinvasiven Einsatz des Instruments 10 möglicherweise nicht durch einen im Querschnitt kleinen Zugang entfernt werden können, kann das Instrument 10 in die in den Figuren 9 und 10 dargestellte Clipentnahmestellung überführt werden. Hierzu wird ausgehend von der Clipspreizstellung das zweite Betätigungsglied 42 wieder vom ersten Betätigungsglied 36 weg verschwenkt, und zwar so weit, bis die Seitenfläche 144 an der Endanschlagfläche 142 anschlägt. Der Nocken 76 nimmt dann wiederum eine Zwischenstellung in den Führungsschlitzen 86 ein und die Anlageflächen 96 liegen flächig aneinander an. Der Clip 12 wird beim Übergang in die Clipentnahmestellung wieder geschlossen, das heißt seine Klemmarme liegen wieder paarweise aneinander an. Die Clipaufnahme 112 ist proximalseitig und distalseitig durch die proximalen und distalen Clipaufnahmeanschläge 72 beziehungsweise 118 geschlossen. Ein seitliches Entweichen des Clips 12 aus der Clipaufnahme 112 ist aufgrund der Ränder 110 nicht möglich, so dass der Clip 12, ohne aus der Clipaufnahme 112 herausfallen zu können, aus dem Körper des Patienten herausgeführt werden kann.

Zum Vorbereiten des Instruments 10 zum Entfernen eines weiteren Clips 12 muss die zweite Anschlageinrichtung 126 temporär deaktiviert werden, und zwar durch temporäres Ausschalten des Endanschlags 138, so dass die Betätigungsglieder 36 und 42 wieder in ihre relativ zueinander maximal aufgespreizte oder entfernte Stellung bewegt werden können, wie sie schematisch in Figur 6 dargestellt ist. Dabei öffnet sich die Clipaufnahme 112 durch Verschwenken der Werkzeugelemente 50 voneinander weg, so dass der aus dem Patientenkörper entfernte Clip 12 aus der Clipaufnahme 112 entnommen werden kann.

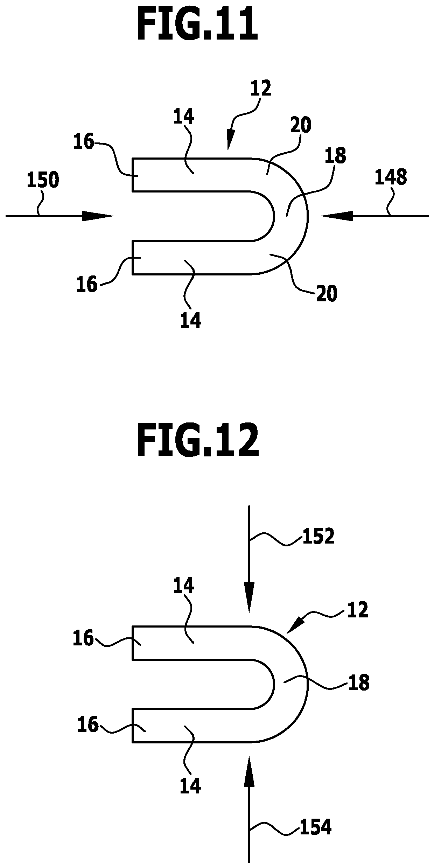

In Figur 11 ist das Zusammenwirken der Spreizeinrichtung 52 mit dem angelegten und zu entfernenden Clip 12 schematisch dargestellt. Der in Figur 12 in Draufsicht dargestellte Clip 12 kann durch Bewegung des proximalen Clipaufnahmeanschlags 72 in Richtung des Pfeils 150, also in distaler Richtung sowie durch Bewegung der distalen Clipaufnahmeanschläge 118 in Richtung des Pfeils 148, also in proximaler Richtung, in der beschriebenen Weise aufgespreizt werden.

Alternativ ist es jedoch auch denkbar, die Spreizeinrichtung 52 derart zu gestalten, dass der Clip 12 nicht zwischen seinen freien Enden 16 und den Verbindungsbereichen 18 in der Clipaufnahme 112 gehalten wird, sondern zwischen den beiden gegenüberliegenden, paarweise aneinander anliegenden Klemmarmen 14. Dies ist schematisch dargestellt in Figur 12, in der der Clip 12 ebenfalls in Draufsicht dargestellt ist. Ein Aufspreizen kann insbesondere erfolgen durch Einführen von zwei aufeinander zu weisenden Spreizgliedern, die ähnlich ausgebildet sind wie die Spreizglieder 100, in Richtung der Pfeile 152 und 154 aufeinander zu, die direkt zwischen die aneinander anliegenden Klemmarme 14 eingreifen und diese voneinander weg aufspreizen.

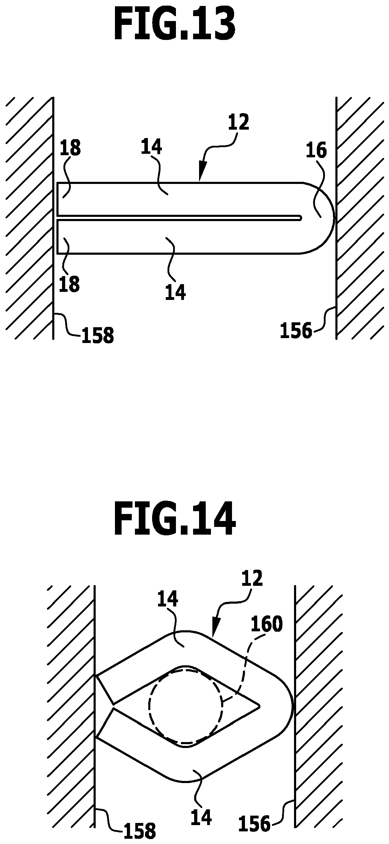

In den Figuren 13 und 14 ist eine alternative, manuelle, nicht erfindungsgemäße Vorgehensweise zum Lösen des Clips 12 von einem Gefäß oder Hohlorgan schematisch dargestellt. Der Clip 12 wird mit seinen freien Enden 16 an einen Klemmbacken 156 und mit den Verbindungsbereichen 18 an einen Klemmbacken 158 herangeführt. Die parallel zueinander verlaufende Klemmbackenflächen aufweisenden Klemmbacken 156 und 158 werden gegeneinander bewegt und der Clip 12 so zwangsweise aufgequetscht, und zwar durch Verformung der Klemmarme 14, so dass diese teilweise voneinander weg bewegt werden und so ein zwischen ihnen ursprünglich geklemmtes Hohlorgan oder Gefäß 160 freigeben.

Das Instrument 10 kann auf einfache Weise gereinigt werden, und zwar indem der Instrumentengriff 26 vom Schaft 28 getrennt wird. Ebenso kann optional auch der Schaft 28 von der Spreizeinrichtung 52 gelöst werden.

Des Weiteren ist es denkbar, den Clip 12 nach Entfernen von einem Gefäß nicht außerhalb des Körpers des Patienten zu übergeben, sondern in diesem an ein anderes geeignetes Instrument, wenn der Clip 12 vom Operationssitus entfernt ist. Mit dem anderen Instrument kann dann der Clip 12 in der Clipaufnahmestellung aus der Clipaufnahme 112 entnommen werden.

## Patentansprüche

1. Chirurgisches Instrument (10) zum Entfernen eines an ein Hohlorgan angelegten chirurgischen Clips (12), welcher zwei Klemmarme (14) aufweist, die zwei freie Enden (16) und zwei miteinander verbundene, einen Verbindungsbereich (18) definierende Enden (20) aufweisen, welches Instrument (10) ein proximales und ein distales Ende (22, 24) aufweist, wobei am distalen Ende (24) eine Spreizeinrichtung (52) zum Fassen und Aufspreizen eines angelegten Clips (12) von einer Anlegestellung in eine Lösestellung angeordnet ist, wobei die Spreizeinrichtung (52) eine Clipaufnahme (112) umfasst und wobei sie von einer Einführstellung, in welcher sie eine minimale Querschnittsfläche definiert, in eine Clipaufnahmestellung überführbar ist, in welcher die Clipaufnahme (112) zur Aufnahme des zu entfernenden Clips (12) geöffnet ist, wobei die Spreizeinrichtung (52) mindestens einen proximalen und mindestens einen distalen Clipaufnahmeanschlag (72, 118) aufweist, welche die Clipaufnahme (112) proximalseitig und distalseitig begrenzen, und wobei zwei distalseitige Clipaufnahmeanschläge (118) vorgesehen sind, **dadurch gekennzeichnet, dass** ein Querabstand (122) zwischen den beiden distalseitigen Clipaufnahmeanschlägen (118) quer zur Längsrichtung (30) beim Übergang von der Clipaufnahmestellung in eine Clipspreizstellung, in welcher der zu entfernende Clip (12) in der Clipaufnahme (112) aufgespreizt haltbar ist, zunimmt und dass ein Abstand (120) in Längsrichtung (30) zwischen dem mindestens einen proximalen und dem mindestens einen distalen Clipaufnahmeanschlag (72, 118) veränderbar ist.

2. Chirurgisches Instrument nach Anspruch 1, **dadurch gekennzeichnet, dass** der Abstand (120) beim Übergang von der Clipaufnahmestellung in die Clipspreizstellung abnimmt.

3. Chirurgisches Instrument nach einem der voranstehenden Ansprüche, **dadurch gekennzeichnet, dass** die Spreizeinrichtung (52) von der Clipspreizstellung in eine Clipentnahmestellung überführbar ist, in welcher der zu entfernende Clip (12) in der Clipaufnahme (112) geschlossen haltbar ist.

4. Chirurgisches Instrument nach Anspruch 3, **dadurch gekennzeichnet, dass** die Clipentnahmestellung und die Einführstellung identisch sind.

5. Chirurgisches Instrument nach einem der voranstehenden Ansprüche, **gekennzeichnet durch** einen langgestreckten Schaft (28), an dessen distalem Ende die Spreizeinrichtung (52) angeordnet ist.

6. Chirurgisches Instrument nach Anspruch 5, **dadurch gekennzeichnet, dass**, dass der mindestens eine distale Clipaufnahmeanschlag (118) relativ zum Schaft (28) um eine Schwenkachse (56) verschwenkbar angeordnet ist, wobei insbesondere die beiden distalen Clipaufnahmeanschläge (118) um eine gemeinsame Schwenkachse (56) relativ zueinander verschwenkbar gelagert sind.

7. Chirurgisches Instrument nach einem der voranstehenden Ansprüche, **dadurch gekennzeichnet, dass** die Spreizeinrichtung (52) zwei relativ zueinander bewegbare Werkzeugelemente (50) umfasst und dass der mindestens eine distale Clipaufnahmeanschlag (118) an den beiden Werkzeugelementen (50) angeordnet oder ausgebildet ist.

8. Chirurgisches Instrument nach Anspruch 7, **dadurch gekennzeichnet, dass** die zwei Werkzeugelemente (50) relativ zum Schaft (28) verschiebbar und/oder verschwenkbar gelagert sind.

9. Chirurgisches Instrument nach Anspruch 7 oder 8, **dadurch gekennzeichnet, dass** die Werkzeugelemente (50) in der Clipspreizstellung eine geöffnete Stellung einnehmen.

10. Chirurgisches Instrument nach einem der voranstehenden Ansprüche, **dadurch gekennzeichnet, dass** die Spreizeinrichtung (52) ein in proximaler Richtung weisendes Spreizglied (100) umfasst, welches insbesondere zwischen zwei Verbindungsbereiche (18) der Klemmarme (14) des zu entfernenden Clips (12) einführbar ist.

11. Chirurgisches Instrument nach Anspruch 10, **dadurch gekennzeichnet, dass** die Spreizeinrichtung (52) zwei relativ zueinander bewegbare Werkzeugelemente (50) umfasst , dass die zwei Werkzeugelemente (50) jeweils ein Spreizgliedteil (102) umfassen und dass die Spreizgliedteile (102) das Spreizglied (100) bilden und dass die Spreizgliedteile (102) in der Einführ- und/oder der Clipentnahmestellung aneinander anliegen zur Ausbildung des Spreizglieds (100).

12. Chirurgisches Instrument nach einem der voranstehenden Ansprüche , **gekennzeichnet durch** eine erste Anschlageinrichtung (124) zum Definieren der Einführstellung.

13. Chirurgisches Instrument nach einem der Ansprüche 3 bis 12, **gekennzeichnet durch** eine zweite Anschlageinrichtung (126) zum Definieren der Clipentnahmestellung.

14. Chirurgisches Instrument nach Anspruch 12 oder 13, **dadurch gekennzeichnet, dass** die erste und/oder die zweite Anschlageinrichtung (124, 126) an der Betätigungseinrichtung (34) angeordnet oder ausgebildet sind.

15. Chirurgisches Instrument nach einem der Ansprüche 12 bis 14, **dadurch gekennzeichnet, dass** die erste und/oder die zweite Anschlageinrichtung (124, 126) ausgebildet sind zum Begrenzen einer Bewegung relativ zueinander bewegbarer Betätigungsglieder (36, 42) der Betätigungseinrichtung (34).

## Claims

1. Surgical instrument (10) for removing a surgical clip (12) applied to a hollow organ, the clip (12) comprising two clamping arms (14) having two free ends (16) and two ends (20) connected to each other and defining a connecting region (18), and the instrument (10) having a proximal and a distal end (22, 24), wherein a spreading device (52) for grasping and spreading apart an applied clip (12) from an applied position to a released position is arranged at the distal end (24), wherein the spreading device (52) comprises a clip receptacle (112), and wherein it is transferable from an insertion position in which it defines a minimal cross-sectional area to a clip receiving position in which the clip receptacle (112) is open to receive the clip (12) to be removed, wherein the spreading device (52) comprises at least one proximal and at least one distal clip receiving stop (72, 118), which delimit the clip receptacle (112) at the proximal side and at the distal side, and wherein two clip receiving stops (118) are provided at the distal side, **characterized in that** a transverse spacing (122) between the two clip receiving stops (118) at the distal side increases transversely to the longitudinal direction (30) during transfer from the clip receiving position to a clip spreading position in which the clip (12) to be removed can be held spread apart in the clip receptacle (112) and **in that** a spacing (120) in the longitudinal direction (30) between the at least one proximal and the at least one distal clip receiving stop (72, 118) is alterable.

2. Surgical instrument in accordance with claim 1, **characterized in that** the spacing (120) decreases during transfer from the clip receiving position to the clip spreading position.

3. Surgical instrument in accordance with any one of the preceding claims, **characterized in that** the spreading device (52) is transferable from the clip spreading position to a clip removal position in which the clip (12) to be removed can be held closed in the clip receptacle (112).

4. Surgical instrument in accordance with claim 3, **characterized in that** the clip removal position and the insertion position are identical.

5. Surgical instrument in accordance with any one of the preceding claims, **characterized by** an elongate shaft (28), at the distal end of which the spreading device (52) is arranged

6. Surgical instrument in accordance with claim 5, **characterized in that** the at least one distal clip receiving stop (118) is arranged so as to be pivotable about a pivot axis (56) relative to the shaft (28),
wherein, in particular, the two distal clip receiving stops (118) are mounted so as to be pivotable about a common pivot axis (56) relative to each other.

7. Surgical instrument in accordance with any one of the preceding claims, **characterized in that** the spreading device (52) comprises two tool elements (50) movable relative to each other, and **in that** the at least one distal clip receiving stop (118) is arranged or formed on the two tool elements (50).

8. Surgical instrument in accordance with claim 7, **characterized in that** the two tool elements (50) are mounted so as to be displaceable and/or pivotable relative to the shaft (28).

9. Surgical instrument in accordance with claims 7 or 8, **characterized in that** the tool elements (50) in the clip spreading position assume an open position..

10. Surgical instrument in accordance with any one of the preceding claims, **characterized in that** the spreading device (52) comprises a spreading member (100) facing in the proximal direction, which, in particular, is insertable between two connecting regions (18) of the clamping arms (14) of the clip (12) to be removed.

11. Surgical instrument in accordance with claim 10, **characterized in that** the spreading device (52) comprises two tool elements (50) movable relative to each other, and **in that** the two tool elements (50) each comprise a spreading member part (102), and **in that** the spreading member parts (102) form the spreading member (100), and **in that** the spreading member parts (102) in the insertion and/or the clip removal position lie against each other so as to form the spreading member (100).

12. Surgical instrument in accordance with any one of the preceding claims, **characterized by** a first stop device (124) for defining the insertion position.

13. Surgical instrument in accordance with any one of claims 3 to 12, **characterized by** a second stop device (126) for defining the clip removal position.

14. Surgical instrument in accordance with claim 12 or 13, **characterized in that** the first and/or the second stop device (124, 126) is/are arranged or formed on the actuating device (34).

15. Surgical instrument in accordance with any one of claims 12 to 14, **characterized in that** the first and/or the second stop device (124, 126) is/are configured to delimit a movement of actuating members (36, 42) of the actuating device (34), which are movable relative to each other.

## Revendications

1. Instrument chirurgical (10) destiné à retirer un clip chirurgical (12), qui est posé sur un organe creux et présente deux bras de serrage (14) présentant deux extrémités libres (16) et deux extrémités (20) reliées l'une à l'autre en définissant une zone de liaison (18), instrument (10) comportant une extrémité proximale et une extrémité distale (22, 24), et
dans lequel à l'extrémité distale (24) est agencé un dispositif d'écartement (52) destiné à saisir et produire l'ouverture par écartement d'un clip (12) à partir d'une position posée jusqu'à une position de retrait,
dans lequel le dispositif d'écartement (52) comprend un logement d'accueil de clip (112) et dans lequel il peut être transféré d'une position d'insertion, dans laquelle il définit une surface de section transversale minimale, à une position d'accueil de clip, dans laquelle le logement d'accueil de clip (112) est ouvert pour accueillir le clip (12) à retirer,
dans lequel le dispositif d'écartement (52) présente au moins une butée proximale de logement d'accueil de clip et au moins une butée distale de logement d'accueil de clip (72, 118), qui délimitent le logement d'accueil de clip (112) du côté proximal et du côté distal,
et dans lequel sont prévues deux butées de logement d'accueil de clip (118) du côté distal,
**caractérisé en ce qu'**une distance transversale (122) entre les deux butées de logement d'accueil de clip (118) du côté distal, transversalement à la direction longitudinale (30), augmente lors du passage de la position d'accueil de clip à une position d'écartement de clip dans laquelle le clip (12) à retirer peut être maintenu écarté dans le logement d'accueil de clip (112), et **en ce qu'**une distance (120) dans la direction longitudinale (30) entre ladite au moins une butée proximale et ladite au moins une butée distale de logement d'accueil de clip (72, 118), peut varier.

2. Instrument chirurgical selon la revendication 1,
**caractérisé en ce que** ladite distance (120) diminue lors du passage de la position d'accueil de clip à la position d'écartement de clip.

3. Instrument chirurgical selon l'une des revendications précédentes, **caractérisé en ce que** le dispositif d'écartement (52) peut être transféré de la position d'écartement de clip à une position de prélèvement de clip, dans laquelle le clip (12) à retirer peut être maintenu fermé dans le logement d'accueil de clip (112).

4. Instrument chirurgical selon la revendication 3,
**caractérisé en ce que** la position de prélèvement de clip et la position d'insertion sont identiques.

5. Instrument chirurgical selon l'une des revendications précédentes, **caractérisé par** un corps allongé (28) à l'extrémité distale duquel est agencé le dispositif d'écartement (52).

6. Instrument chirurgical selon la revendication 5,
**caractérisé en ce que** ladite au moins une butée distale de logement d'accueil de clip (118) est agencée de manière à pouvoir pivoter par rapport au corps allongé (28), autour d'un axe de pivotement (56),
lesdites deux butées distales de logement d'accueil de clip (118) étant notamment montées de manière à pouvoir pivoter l'une par rapport à l'autre autour d'un axe de pivotement commun (56).

7. Instrument chirurgical selon l'une des revendications précédentes, **caractérisé en ce que** le dispositif d'écartement (52) comporte deux éléments d'outil (50) mobiles l'un par rapport à l'autre, et **en ce que** ladite au moins une butée distale de logement d'accueil de clip (118) est agencée ou formée sur lesdits deux éléments d'outil (50).

8. Instrument chirurgical selon la revendication 7,
**caractérisé en ce que** lesdits deux éléments d'outil (50) sont montés de manière coulissante et/ou pivotante par rapport au corps allongé (28).

9. Instrument chirurgical selon la revendication 7 ou la revendication 8, **caractérisé en ce que** lesdits éléments d'outil (50) prennent une position d'ouverture dans la position d'écartement de clip.

10. Instrument chirurgical selon l'une des revendications précédentes, **caractérisé en ce que** le dispositif d'écartement (52) comprend un organe d'écartement (100) dirigé en direction proximale, qui peut notamment être introduit entre deux zones de liaison (18) des bras de serrage (14) du clip (12) à retirer.

11. Instrument chirurgical selon la revendication 10,
**caractérisé en ce que** le dispositif d'écartement (52) comporte deux éléments d'outil (50) mobiles l'un par rapport à l'autre, **en ce que** les deux éléments d'outil (50) comportent respectivement une partie d'organe d'écartement (102), et **en ce que** les parties d'organe d'écartement (102) forment l'organe d'écartement (100), et **en ce que** les parties d'organes d'écartement (102) s'appuient l'une contre l'autre dans la position d'insertion et/ou la position de prélèvement de clip, pour former l'organe d'écartement (100).

12. Instrument chirurgical selon l'une des revendications précédentes, **caractérisé par** un premier système de butée (124) pour définir la position d'insertion.

13. Instrument chirurgical selon l'une des revendications 3 à 12, **caractérisé par** un deuxième système de butée (126) pour définir la position de prélèvement de clip.

14. Instrument chirurgical selon la revendication 12 ou la revendication 13, **caractérisé en ce que** le premier et/ou le deuxième système de butée (124, 126) sont agencés ou formés sur le dispositif d'actionnement (34).

15. Instrument chirurgical selon l'une des revendications 12 à 14, **caractérisé en ce que** le premier et/ou le deuxième système de butée (124, 126) sont conçus pour limiter un mouvement de l'un par rapport à l'autre d'organes d'actionnement (36, 42) mobiles l'un par rapport à l'autre, du dispositif d'actionnement (34).
